(19) Europäisches Patentamt / European Patent Office / Office européen des brevets

(11) **EP 1 839 567 A1**

(12) **EUROPEAN PATENT APPLICATION**
published in accordance with Art. 158(3) EPC

(43) Date of publication:
**03.10.2007 Bulletin 2007/40**

(51) Int Cl.:
**A61B 5/00** (2006.01)

(21) Application number: **06711491.8**

(22) Date of filing: **10.01.2006**

(86) International application number:
**PCT/JP2006/300121**

(87) International publication number:
**WO 2006/077750 (27.07.2006 Gazette 2006/30)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.01.2005 JP 2005011906**

(71) Applicant: **SYSMEX CORPORATION**
**Kobe-shi,**
**Hyogo 651-0073 (JP)**

(72) Inventors:
• **SAWA, Kenichi**
  **1-chome, Chuo-ku, Kobe-shi, Hyogo, 6510 (JP)**
• **MAEKAWA, Yasunori**
  **1-chome, Chuo-ku, Kobe-shi, Hyogo, 6510 (JP)**
• **ASAKURA, Yoshihiro**
  **1-chome, Chuo-ku, Kobe-shi, Hyogo, 6510 (JP)**

(74) Representative: **HOFFMANN EITLE**
**Patent- und Rechtsanwälte**
**Arabellastrasse 4**
**81925 München (DE)**

(54) **ANALYZER AND CARTRIDGE FOR EXTRACTING ANALYTE**

(57) An analyzer (a blood glucose meter), which shows minor measurement errors even in the case of conducting the extraction and detection of an analyte in parallel, can be obtained. This blood glucose meter (100) comprises a sensor unit (26) having a measurement face (26a) to which glucose extracted from a subject is supplied, a light-receiving element (34) outputting a signal based on the glucose supplied to the measurement face, a sensor contact face (23a) to be in contact with the measurement face and a skin contact face (23b) to be in contact with the skin of the subject, as well as a mesh sheet (23) for supplying the glucose from the skin contact face to the measurement face. The mesh sheet is designed so that the distance between the measurement face and the skin is maintained substantially at a constant level during the contact with the skin.

FIG.5

## Description

Technical Field

The present invention relates to an analyzer and a cartridge for extracting an analyte, and more particularly, it relates to an analyzer and a cartridge extracting an analyte from an organism.

Background Art

An analyzer extracting an analyte (glucose) from an organism without hurting the skin is known in general. An iontophoretic collector formed by arranging a hydrogel pad of a conductive medium, an iontophoretic electrode and a sensor electrode to be in contact with each other is known as such a conventional analyzer. Such an iontophoretic collector is disclosed in International Patent Laying-Open No. WO96/00110, for example.

This iontophoretic collector disclosed in International Patent Laying-Open No. WO96/00110 extracts glucose from an organism by bringing the hydrogel pad into contact with the skin of his/her wrist and supplying a current to the organism through the iontophoretic electrode. Then, the iontophoretic collector calculates the concentration of glucose by detecting electric charge resulting from reaction of the extracted glucose through catalytic action of an enzyme contained in the hydrogel with the sensor electrode. In this iontophoretic collector disclosed in International Patent Laying-Open No. WO96/00110, the hydrogel pad is arranged between the sensor electrode and the skin, whereby the distance between the sensor electrode and the skin substantially coincides with the thickness of the hydrogel pad. Further, International Patent Laying-Open No. WO96/00110 also proposes a technique of employing a suction material such as a sponge in which an ion-conductive medium is absorbed in place of the hydrogel pad. In this case, the distance between the sensor electrode and the skin substantially coincides with the thickness of the suction material such as a sponge.

In the aforementioned iontophoretic collector disclosed in International Patent Laying-Open No. WO96/00110, however, the hydrogel pad or the sponge is so remarkably deformed by external force that the hydrogel pad or the sponge is deformed when compressive force or tensile force is applied from the skin or from outside to the hydrogel pad or the sponge during extraction of glucose, to disperse the distance between the skin and the sensor electrode. Thus, the time in which the extracted glucose reaches the enzyme or the time in which the electric charge resulting from reaction of the extracted glucose reaches the sensor electrode is so dispersed that a measurement error may disadvantageously result if the extraction of glucose and the detection of electric charge are performed in parallel with each other.

Disclosure of the Invention

The present invention has been proposed in order to solve the aforementioned problem, and an object of the present invention is to provide an analyzer and a cartridge for extracting an analyte exhibiting a small number of measurement errors also when performing extraction and detection of an analyte in parallel with each other.

In order to attain the aforementioned object, an analyzer according to a first aspect of the present invention is an analyzer for extracting an analyte from a subject and analyzing the analyte, comprising a sensor member including a supplied face supplied with the analyte extracted from the subject or a product resulting from reaction of the analyte, signal output means outputting a signal based on the analyte or the product supplied to the supplied face and a liquid holding member provided with a first face coming into contact with the supplied face and a second face coming into contact with the skin of the subject and capable of holding a liquid for supplying the analyte or the product from the second face to the supplied face, and the liquid holding member is so formed as to keep the distance between the supplied face and the skin substantially constant while the same is in contact with the skin.

In this analyzer according to the first aspect, as hereinabove described, the liquid holding member capable of holding the liquid for supplying the analyte or the product from the second face to the supplied face is provided and the liquid holding member is so formed as to keep the distance between the supplied face and the skin substantially constant while the same is in contact with the skin, whereby the distance between the supplied face of the sensor member and the skin at the time of extracting the analyte can be kept substantially constant. Therefore, the distance between the supplied face of the sensor member and the skin remains substantially unchanged, and the time up to arrival of the analyte or the product at the sensor member can be kept substantially constant. Thus, occurrence of a measurement error resulting from dispersion in the distance between the skin and the sensor member can be suppressed also when performing extraction and detection of the analyte in parallel with each other.

In the aforementioned analyzer according to the first aspect, the signal output means preferably includes a light source applying light to the sensor member and a photodetector detecting the light passing through the sensor member. According to this structure, the signal output means can easily output the signal by detecting the light passing through the sensor member with the photodetector of the signal output means.

In the aforementioned analyzer according to the first aspect, the signal output means preferably outputs the signal based on the analyte or the product in parallel with the supply of the analyte or the product to the supplied face. According to this structure, the analyzer can output the signal with the signal output means while extracting the analyte, whereby the time from start of extraction of the analyte up to calculation of the analytical result of the analyte can be reduced.

The aforementioned analyzer according to the first aspect preferably further comprises an electrode so arranged as to come into contact with the first face of the liquid holding member and a power source connected to the electrode. According to this structure, the electrode connected with the power source can supply an electric field to the skin of the subject through the liquid holding member, whereby the analyte extracted from the skin of the subject can be moved toward the electrode. Thus, the analyte can be more efficiently extracted from the skin of the subject.

In the aforementioned analyzer according to the first aspect, the liquid holding member is preferably provided in the form of a sheet. According to this structure, the distance between the skin and the sensor member can be reduced, whereby the time from extraction of the analyte up to arrival at the supplied face of the sensor member can be reduced. Thus, the time from start of extraction of the analyte up to calculation of the analytical result of the analyte can be further reduced.

In the aforementioned analyzer according to the first aspect, the liquid holding member preferably keeps a substantially constant thickness when held between the supplied face and the skin. According to this structure, the distance between the supplied face of the sensor member and the skin at the time of extracting the analyte can be easily kept substantially constant.

In the aforementioned analyzer according to the first aspect, the liquid holding member is preferably made of nylon. When employing the liquid holding member of nylon in this manner, the liquid can be reliably held since nylon is excellent in hydrophilicity. Further, nylon is substantially unstretchable, whereby a liquid holding member hardly changing the distance between a surface closer to the sensor and a surface closer to the skin can be easily formed.

In the aforementioned analyzer according to the first aspect, the liquid holding member preferably has a thickness of not more than 1 mm. When employing the liquid holding member having the thickness of not more than 1 mm in this manner, the time up to arrival of the analyte extracted from the skin of the subject at the sensor member can be reduced as compared with a case of employing a liquid holding member having a thickness greater than 1 mm, whereby the time from extraction of the analyte up to detection of the analyte or the product with the sensor member can be reduced. Thus, the time necessary for calculating the analytical result of the analyte after starting extraction of the analyte can be reduced.

In the aforementioned analyzer according to the first aspect, the liquid holding member preferably includes a plurality of through-holes passing through the first face and the second face. According to this structure, the plurality of through-holes passing through the first face and the second face can hold the liquid, whereby the analyte extracted from the subject or the product can be easily made to reach the supplied face.

A cartridge for extracting an analyte according to a second aspect of the present invention is a cartridge for extracting an analyte attachable to an analyzer extracting an analyte from a subject and analyzing the analyte, comprising a sensor member including a supplied face supplied with the analyte extracted from the subject or a product resulting from reaction of the analyte and a liquid holding member provided with a first face coming into contact with the supplied face and a second face coming into contact with the skin of the subject and capable of holding a liquid for supplying the analyte or the product from the second face to the supplied face, and the liquid holding member is so formed as to keep the distance between the supplied face and the skin substantially constant while the same is in contact with the skin.

In this cartridge for extracting an analyte according to the second aspect, as hereinabove described, the liquid holding member capable of holding the liquid for supplying the analyte or the product from the second face to the supplied face is provided and the liquid holding member is so formed as to keep the distance between the supplied face and the skin substantially constant while the same is in contact with the skin, whereby the distance between the supplied face of the sensor member and the skin at the time of extracting the analyte can be kept substantially constant. Therefore, the distance between the supplied face of the sensor member and the skin remains substantially unchanged, and the time up to arrival of the analyte or the product at the sensor member can be kept substantially constant. Thus, occurrence of a measurement error resulting from dispersion in the distance between the skin and the sensor member can be suppressed also when performing extraction and detection of the analyte in parallel with each other.

In the aforementioned cartridge for extracting an analyte according to the second aspect, the sensor member preferably contains a first enzyme serving as a catalyst for the analyte in the supplied face. According to this structure, the distance between the skin and the first enzyme can be kept substantially constant, whereby occurrence of a measurement error can be further suppressed.

In this case, the sensor member preferably further contains a second enzyme serving as a catalyst for a first substance produced from the analyte in the supplied face. According to this structure, the distance between the skin and the second enzyme can be kept substantially constant, whereby occurrence of a measurement error can be further suppressed.

In the aforementioned structure including the substance produced from the analyte, the sensor member preferably further contains a chromogenic dye reacting with a second substance produced from the first substance. According to this structure, the distance between the skin and the chromogenic dye can be kept substantially constant, whereby occurrence of a measurement error can be further suppressed.

In the aforementioned cartridge for extracting an analyte according to the second aspect, the liquid holding member is preferably provided in the form of a sheet. According to this structure, the distance between the skin and the sensor member can be reduced, whereby the time from extraction of the analyte up to detection of the analyte or the product with the sensor member can be reduced. Thus, the time from start of extraction of the analyte up to calculation of the analytical result of the analyte can be further reduced.

In the aforementioned cartridge for extracting an analyte according to the second aspect, the liquid holding member preferably keeps a substantially constant thickness when held between the supplied face and the skin. According to this structure, the distance between the supplied face of the sensor member and the skin at the time of extracting the analyte can be easily kept substantially constant.

In the aforementioned cartridge for extracting an analyte according to the second aspect, the liquid holding member is preferably made of nylon. When employing the liquid holding member of nylon in this manner, the liquid can be reliably held since nylon is excellent in hydrophilicity. Further, nylon is substantially unstretchable, whereby a liquid holding member hardly changing the distance between a surface closer to the sensor and a surface closer to the skin can be easily formed.

In the aforementioned cartridge for extracting an analyte according to the second aspect, the liquid holding member preferably has a thickness of not more than 1 mm. When employing the liquid holding member having the thickness of not more than 1 mm in this manner, the time up to arrival of the analyte extracted from the skin of the subject at the sensor member can be reduced as compared with a case of employing a liquid holding member having a thickness greater than 1 mm, whereby the time from extraction of the analyte up to detection of the analyte or the product with the sensor member can be reduced. Thus, the time necessary for calculating the analytical result of the analyte after starting extraction of the analyte can be reduced.

In the aforementioned cartridge for extracting an analyte according to the second aspect, the liquid holding member preferably includes a plurality of through-holes passing through the first face and the second face. According to this structure, the plurality of through-holes passing through the first face and the second face can hold the liquid, whereby the analyte extracted from the subject or the product can be easily made to reach the supplied face.

In this case, the plurality of through-holes of the liquid holding member may be arranged in the form of a lattice.

The aforementioned cartridge for extracting an analyte according to the second aspect preferably further comprises an electrode arranged to be held between the sensor member and the liquid holding member. According to this structure, an electric field can be easily supplied to the skin of the subject through the liquid holding member by simply connecting the electrode to the power source, whereby the analyte extracted from the skin of the subject can be moved toward the electrode. Thus, the analyte can be more efficiently extracted from the skin of the subject.

In the aforementioned cartridge for extracting an analyte according to the second aspect, the liquid holding member is preferably so arranged as to cover the whole of the supplied face. According to this structure, the analyte supplied to the sensor member through the liquid holding member can be detected through the overall supplied face, whereby the analyte can be more precisely analyzed.

Brief Description of the Drawings

[Fig. 1] A perspective view showing a state attaching a blood glucose meter mounted with an extraction cartridge according to an embodiment of the present invention to the wrist of a subject.

[Fig. 2] A plan view showing the internal structure of the blood glucose meter shown in Fig. 1.

[Fig. 3] A sectional view taken along the line 300-300 in Fig. 2.

[Fig. 4] A sectional view taken along the line 400-400 in Fig. 2.

[Fig. 5] An enlarged diagram showing the state attaching the blood glucose meter shown in Fig. 3 to the wrist of the subject.

[Fig. 6] A plan view showing the structure of the extraction cartridge attached to the blood glucose meter shown in Fig. 2.

[Fig. 7] A schematic diagram showing the structure of a sensing portion of the blood glucose meter shown in Fig. 1.

[Fig. 8] A perspective view showing an extraction cartridge set storing the extraction cartridge according to the embodiment of the present invention.

[Fig. 9] A sectional view taken along the line 500-500 in Fig. 8.

[Fig. 10] A perspective view showing a state of opening the extraction cartridge set shown in Fig. 8.

[Fig. 11] A plan view of the extraction cartridge set shown in Fig. 10.

[Fig. 12] A perspective view showing a microneedle employed for pretreatment.

[Fig. 13] A sectional view showing a state of the skin subjected to the pretreatment with the microneedle shown in Fig. 12.

[Fig. 14] A flow chart showing an operation procedure of blood glucose measurement with the blood glucose meter shown in Fig. 1.

[Fig. 15] A sectional view for illustrating an operation for taking out the extraction cartridge from the extraction cartridge set shown in Fig. 8.

[Fig. 16] A sectional view for illustrating the operation for taking out the extraction cartridge from the extraction cartridge set shown in Fig. 8.

[Fig. 17] A sectional view for illustrating the operation for taking out the extraction cartridge from the extraction cartridge set shown in Fig. 8.

[Fig. 18] A sectional view for illustrating the operation for taking out the extraction cartridge from the extraction cartridge set shown in Fig. 8.

[Fig. 19] A perspective view for illustrating the operation for taking out the extraction cartridge from the extraction cartridge set shown in Fig. 8.

[Fig. 20] A correlation diagram showing the relation between the quantities of pure water contained in liquid supply members of the extraction cartridge set shown in Fig. 8 and the quantities of pure water transferred from the liquid supply members to mesh sheets of the extraction cartridge.

[Fig. 21] A model diagram for illustrating the principle of glucose extraction with the blood glucose meter shown in Fig. 1.

[Fig. 22] A model diagram for illustrating the principle of glucose extraction with the blood glucose meter shown in Fig. 1.

[Fig. 23] A model diagram for illustrating the principle of glucose extraction with the blood glucose meter shown in Fig. 1.

[Fig. 24] A model diagram for illustrating the principle of glucose extraction with the blood glucose meter shown in Fig. 1.

[Fig. 25] A plan view showing the internal structure of a blood glucose meter mounted with an extraction cartridge according to a modification of the embodiment. Best Modes for Carrying Out the Invention

An embodiment of the present invention is now described with reference to the drawings.

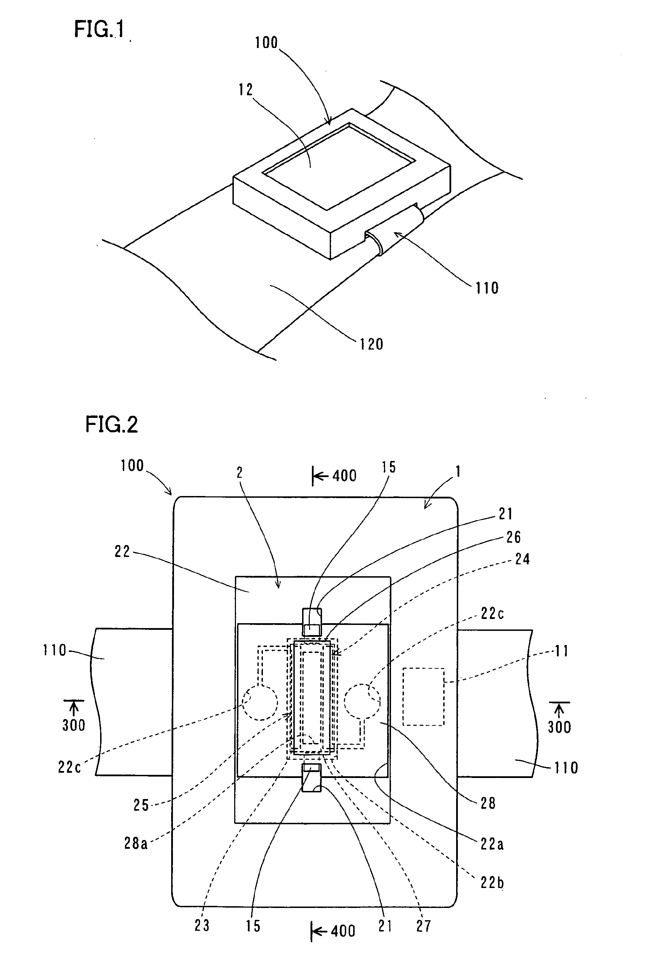

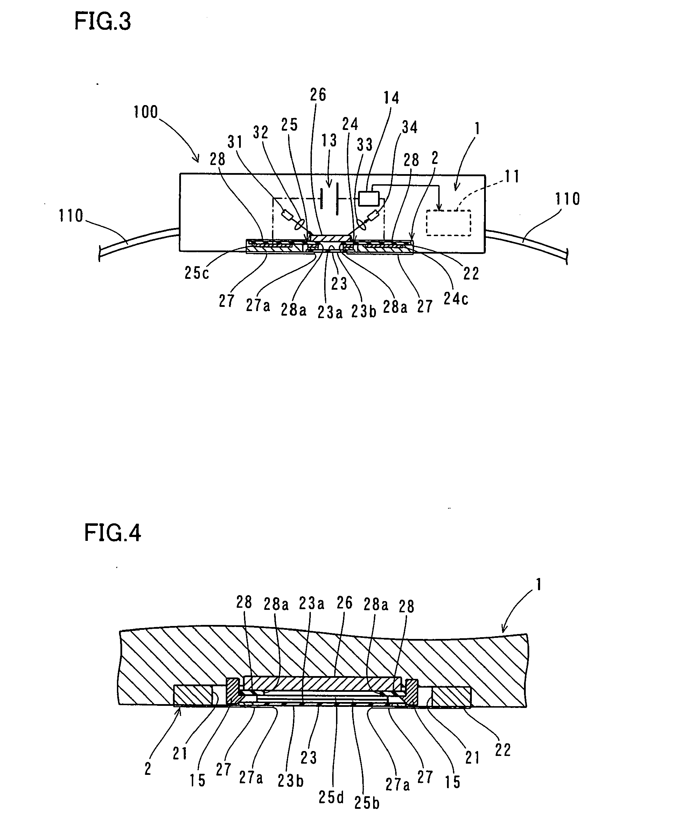

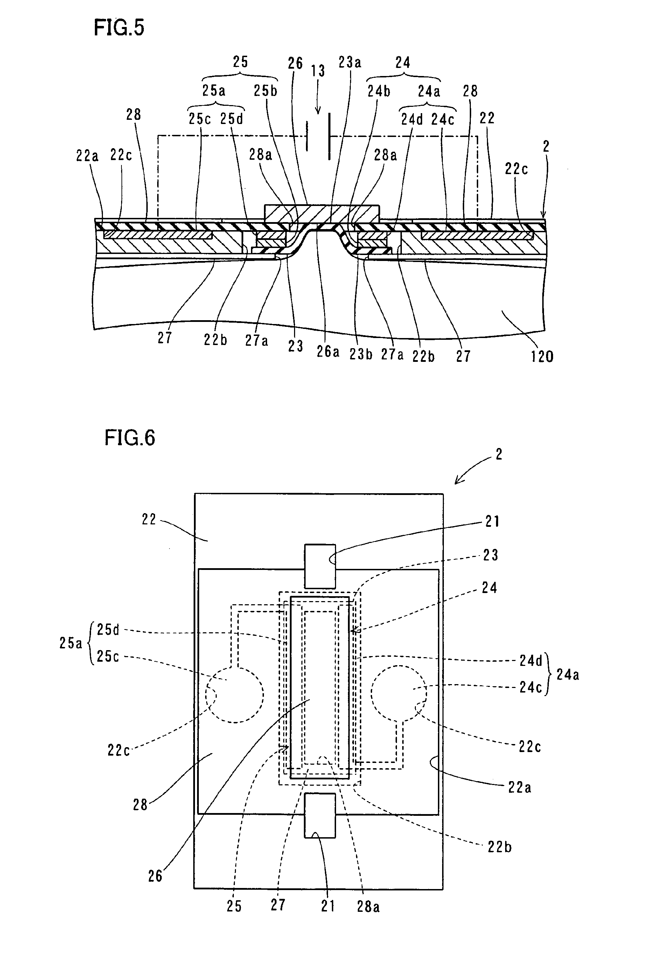

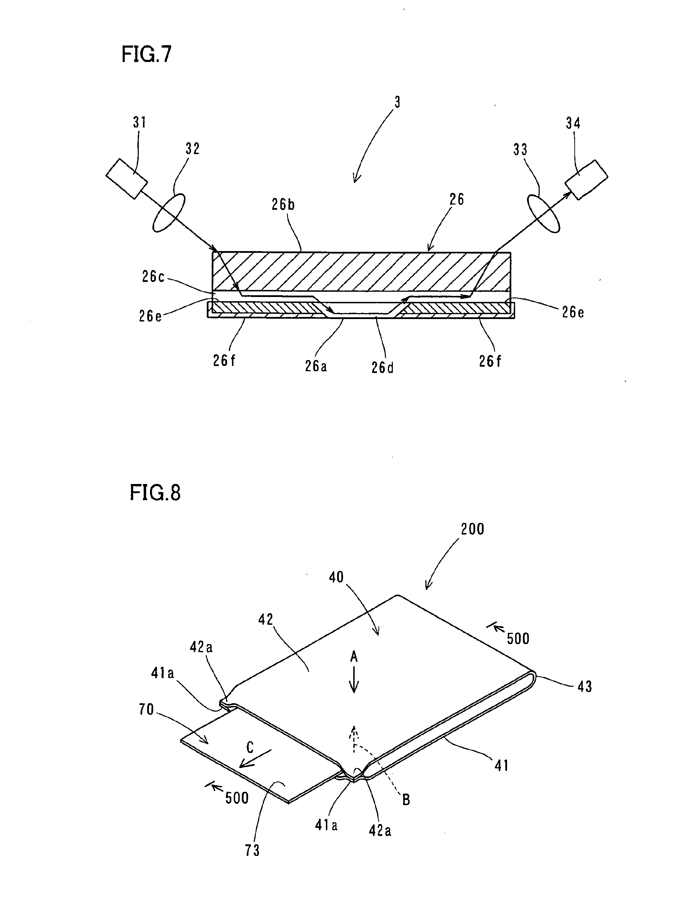

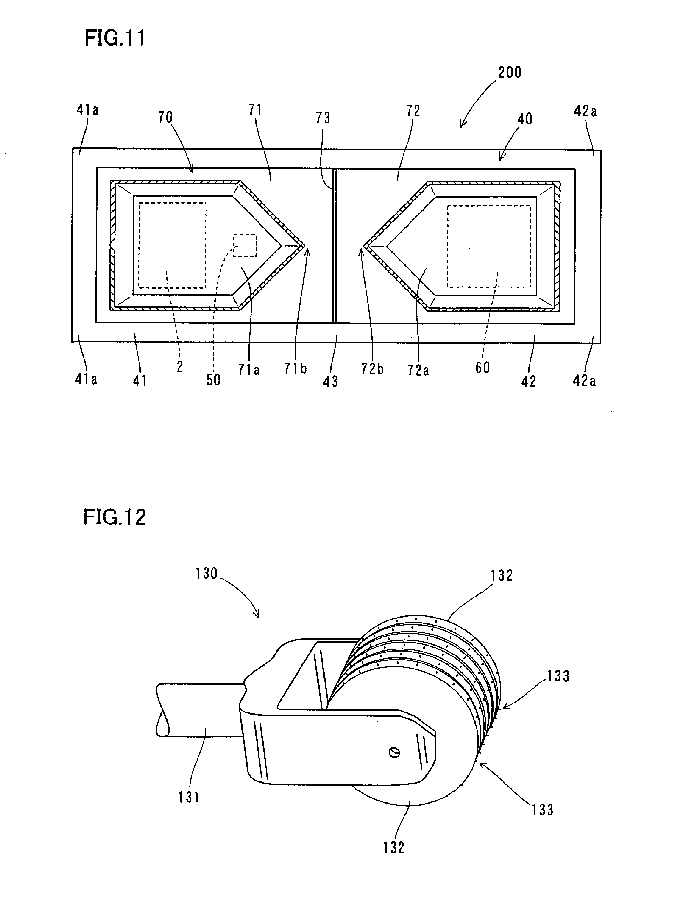

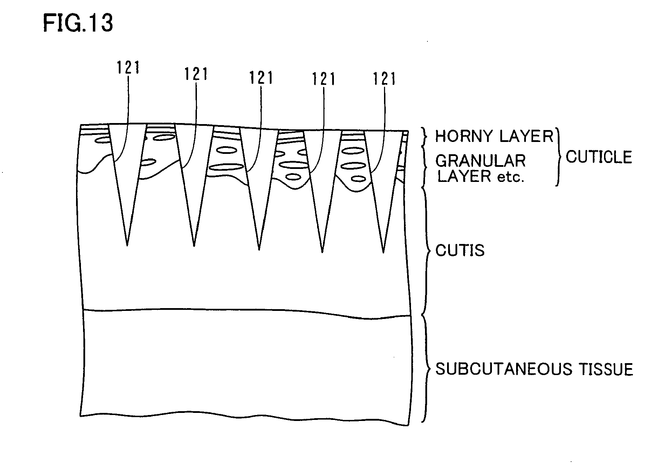

Fig. 1 is a perspective view showing a state attaching a blood glucose meter mounted with an extraction cartridge according to an embodiment of the present invention to the wrist of a subject. Figs. 2 to 4 are diagrams showing the internal structure of the blood glucose meter shown in Fig. 1. Fig. 5 is an enlarged diagram showing the state attaching the blood glucose meter shown in Fig. 3 to the wrist of the subject. Fig. 6 is a plan view showing the structure of the extraction cartridge attached to the blood glucose meter shown in Fig. 2. Fig. 7 is a schematic diagram showing the structure of a sensing portion of the blood glucose meter shown in Fig. 1. Figs. 8 to 11 are perspective views showing an extraction cartridge set storing the extraction cartridge according to the embodiment of the present invention. Fig. 12 is a perspective view showing a microneedle employed for pretreatment. Fig. 13 is a sectional view showing a state of the skin subjected to the pretreatment with the microneedle shown in Fig. 12. First, the overall structure of a blood glucose meter 100 mounted with an extraction cartridge 2 according to the embodiment of the present invention is described with reference to Figs. 1 to 7, 12 and 13.

The blood glucose meter 100 according to the embodiment of the present invention is an apparatus extracting glucose which is one of biochemical components from an organism and calculating a blood glucose level by analyzing the extracted glucose. This blood glucose meter 100 is formed to be attached to a wrist 120 of a subject with a band member 110, as shown in Fig. 1. A fixture (not shown) having an opening on a prescribed position is mounted on the band member 110. The subject performs pretreatment on an extraction region of the wrist 120 of the subject with a needle roller 130 (see Fig. 12) through the opening of the fixture.

The needle roller 130 is constituted of an arm 131 and a plurality of rollers 132 rotatably supported on the arm 132, as shown in Fig. 12. A plurality of minute needles 133 are formed on the outer peripheral surfaces of these rollers 132 at a prescribed interval. These needles 133 have such a quantity of protrusion (about 0.3 mm) that the same can pass through the cuticle including a horny layer without reaching subcutaneous tissue. When employing this needle roller 130, minute extraction holes 121 passing through the cuticle and reaching the cutis without reaching the subcutaneous tissue can be formed in the skin as shown in Fig. 13, whereby body fluid can be extracted from the organism through the plurality of extraction holes 121. Thus, pain felt by the subject can be eased when extracting glucose from the organism with the blood glucose meter 100 shown in Fig. 1. In this embodiment, Dermaroller by Top-Rol, for example, is used as the needle roller 130.

The blood glucose meter 100 comprises an analytic unit 1 serving as a meter body and an extraction cartridge 2 detachably held on the analytic unit 1, as shown in Figs. 2 and 3. The analytic unit 1 includes a control portion 11, a display portion 12 (see Fig. 1), a DC-system constant-voltage power source 13 (see Fig. 3), an ammeter 14 (see Fig. 3) measuring a current fed from the constant-voltage power source 13 and outputting the result of this measurement to the control portion 11 and two engaging hooks 15, as shown in Figs. 2 and 3. Further, the analytic unit 1 is provided with a monochromatic light source 31, a lens 32, another lens 33 and a photoreceptor 34 constituting a sensing portion 3 shown in Fig. 7. The control portion 11 is constituted of a CPU, a ROM, a RAM and the like, and has a function for calculating the quantity of glucose and thereafter calculating the blood glucose level from the calculated quantity of glucose. The display portion 12 is provided for displaying the quantity of glucose and the blood glucose level calculated by the control portion 11.

The constant-voltage power source 13 is connected to terminal portions 24c and 25c of an anode 24 and a cathode 25 of the extraction cartridge 2 described later, as shown in Figs. 3 and 5. This constant-voltage power source 13 applies a voltage of 0.8 V between the anode 24 and the cathode 25. The two engaging hooks 15 are provided for fixing the extraction cartridge 2 to the analytic unit 1, as shown in Figs. 2 and 4. The monochromatic light source 31 and the lens 32 constituting the sensing portion 3 provided on the analytic unit 1 have a function for supplying light for analysis to a sensor member 26. described later, as shown in Figs. 3 and 7. The sensing portion 3 is so formed that light passing through the sensor member 26 enters the photoreceptor 34 through the lens 33.

The extraction cartridge 2 is detachably fixed to the analytic unit 1 by engaging the engaging hooks 15 of the aforementioned analytic unit 1 with two mounting holes 21 formed in the extraction cartridge 2 respectively, as shown in Figs. 2 and 4. This extraction cartridge 2 is so formed that the same can be exchanged and used every measurement of the blood glucose level.

According to this embodiment, the extraction cartridge 2 includes a cartridge body 22 composed of resin of acrylic, a mesh sheet 23 functioning as a medium holding pure water, the anode 24 and the cathode 25, the sensor member 26 constituting the sensing portion 3 (see Fig. 7) for sensing glucose, a double-faced adhesive tape 27 and a substantially insulating electrode sheet 28, as shown in Figs. 5 and 6. A quadrangular concave step portion 22a is provided on the cartridge body 22, as shown in Fig. 6. A through-hole 22b reaching the lower surface of the cartridge body 22 is formed in the central portion of the step portion 22a, as shown in Fig. 5. Circular recess portions 22c are formed on positions of the step portion 22a opposed to each other through the through-hole 22b respectively. The terminal portions 24c and 25c of the anode 24 and the cathode 25 described later are fitted into these two recess portions 22c respectively. The mesh sheet 23, activated carbon electrodes 24b and 25b and working portions 24d and 25d are held in the through-hole 22b. The electrode sheet 28 is fitted into the concave step portion 22a, and arranged between the working portions 24d and 25d and the sensor member 26. An opening 28a of about 9 mm by about 3 mm in size is formed in this electrode sheet 28. A measurement face 26a of the sensor member 26 is exposed toward the mesh sheet 23 through the opening 28a of the aforementioned electrode sheet 28.

According to this embodiment, the mesh sheet 23 has a sensor contact face 23a coming into contact with the measurement face 26a of the sensor member 26 when the blood glucose meter 100 is attached to the wrist 120 of the subject and a skin contact face 23b coming into contact with the skin of the wrist 120 of the subject, as shown in Figs. 3 to 5. Further, the mesh sheet 23 is fixed by the double-faced adhesive tape 27, coming into contact with the lower surfaces of the activated carbon electrodes 24b and 25b, having a thickness of about 20 $\mu$m from the lower surface side of the cartridge body 22, as shown in Fig. 5. This double-faced adhesive tape 27 has an opening 27a defining a measurement portion (extraction region) of the skin, and is stuck along the overall lower surface of the cartridge body 22. The mesh sheet 23 is in a state containing pure water at the time of extraction of glucose. Further, the mesh sheet 23 is so formed as to come into contact with the lower surface of the sensor member 26 by being pushed upward by a swell of the wrist 120 of the subject through the opening 27a of the double-faced adhesive tape 27. The mesh sheet 23 used in this embodiment is made of nylon, and has a length of about 10 mm, a width of about 4 mm and a thickness of about 50 $\mu$m. The thickness of the mesh sheet 23 is preferably at least about 1 $\mu$m and not more than about 1 mm (1000 $\mu$m), and more preferably at least about 30 $\mu$m and not more than about 500 $\mu$m. When employing the mesh sheet 23 having a thickness of at least about 30 $\mu$m in this manner, breakage of the mesh sheet 23 resulting from pressing force from the skin at the time of measurement or the like can be easily suppressed.

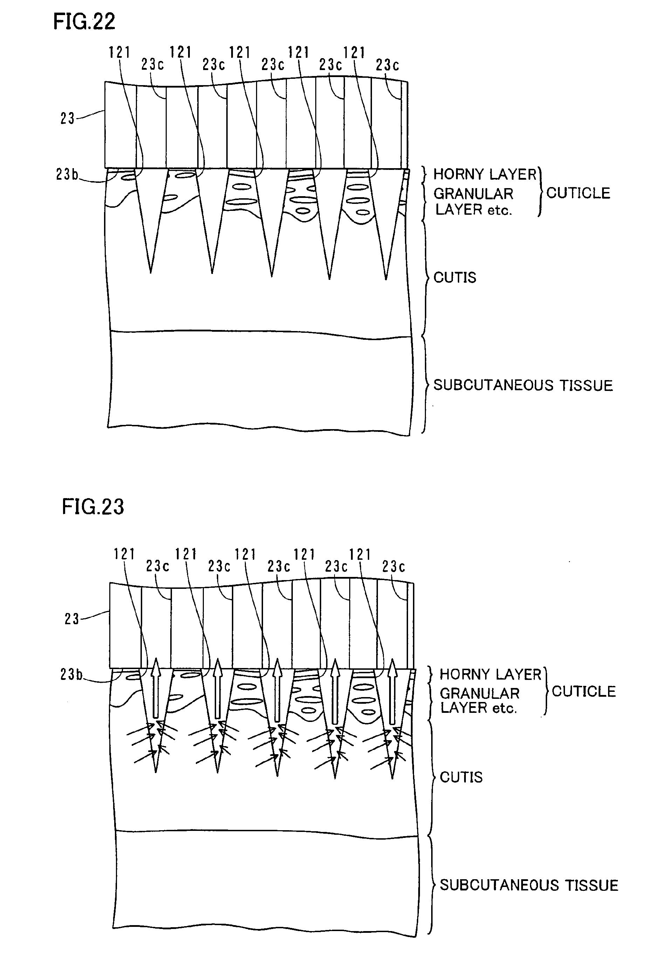

The mesh sheet 23 having the thickness of not more than about 500 $\mu$m is so employed that the time up to arrival of the glucose extracted from the skin of the subject at the measurement face 26a of the sensor member 26 can be reduced as compared with a case of employing a mesh sheet 23 having a thickness greater than about 500 $\mu$m, whereby the time from start of extraction of glucose up to detection of the glucose with the sensor member 26 can be reduced. Thus, the measuring time for the glucose or the blood glucose level can be reduced. This mesh sheet 23 has flexibility while having substantially no stretchability, and is so formed that the thickness thereof remains substantially unchanged. In other words, the mesh sheet 23 is so formed as to have a substantially constant thickness in a state coming into contact with the skin of the wrist 120 of the subject while being held between the measurement face 26a of the sensor member 26 and the skin of the wrist 120 of the subject. Thus, the distance between the skin of the wrist 120 of the subject and the measurement face 26a of the sensor member 26 substantially coincides with the thickness of the mesh sheet 23, and is about 50 $\mu$m according to this embodiment. Further, the mesh sheet 23 is formed by weaving nylon fiber of about 30 $\mu$m in thickness, and has a square network structure of about 33 $\mu$m lengthwise and crosswise respectively. In other words, the mesh sheet 23 has a porous structure having a plurality of through-holes 23c (see Figs. 22 to 24) constituting a mesh. The plurality of through-holes 23c are so formed as to pass through the mesh sheet 23 from the sensor contact face 23a toward the skin contact face 23b. The distance between the skin of the wrist 120 of the subject and the anode 24 and the cathode 25 substantially coincides with the thickness of the mesh sheet 23, and is about 50 $\mu$m according to this embodiment.

The anode 24 and the cathode 25 are constituted of collectors 24a and 25a of silver chloride (AgCl) and the activated carbon electrodes 24b and 25b of activated carbon serving as a porous conductive substance respectively, as shown in Figs. 5 and 7. The collectors 24a and 25a are constituted of the circular terminal portions 24c and 25c fitted into the two recess portions 22c formed in the step portion 22a of the cartridge body 22 respectively and the working portions 24d and 25d connected to the terminal portions 24c and 25c respectively. The activated carbon electrodes 24b and 25b are stuck onto lower portions of the working portions 24d and 25d of the collectors 24a and 25a respectively, and have specific surface areas of about 1000 m$^2$/g to about 3000 m$^2$/g. The terminal portions 24c and 25c of the anode 24 and the cathode 25 are connected to the DC-system constant-voltage power source 13 provided on the analytic unit 1.

The sensor member 26 is arranged on the upper surface of the electrode sheet 28, as shown in Fig. 5. This sensor member 26 has the measurement face 26a performing analysis of glucose on the lower surface side. This measurement face 26a is coated with a mixed gel containing a chromogenic dye pigmenting due to reaction of glucose and prescribed enzymes, and subjected to treatment for drying the mixed gel.

More specifically, the measurement face 26a of the sensor member 26 is coated with a mixed gel prepared by mixing glucose oxidase (GOD) which is oxidase serving as a catalyst for glucose, peroxidase (POD) which is oxidoreductase serving as a catalyst for hydrogen peroxide (H$_2$O$_2$) resulting from reaction of the glucose through catalytic action of GOD and a chromogenic dye pigmenting by reacting with O* (active oxygen) resulting from reaction of H$_2$O$_2$ through catalytic action of POD into a gel, and subjected to treatment for drying the mixed gel.

The sensor member 26 is constituted of a substrate 26b of glass, a first light guide layer 26c mounted on a lower portion of the substrate 26b, a second light guide layer 26d mounted on the center of a lower portion of the first light guide layer 26c, a protective film 26e formed on the lower portion of the first light guide layer 26c to hold the second light guide layer 26d and a light blocking layer 26f covering the outer side of the protective film 26e, as shown in Fig. 7. The first light guide layer 26c has a higher refractive index than the substrate 26b. The second light guide layer 26d has a trapezoidal shape with inclined side portions, and has a higher refractive index than the first light guide layer 26c. The measurement face 26a of the sensor member 26 is a part of the second light guide layer 26d exposed from the protective film 26e, and comes into contact with the upper surface of the mesh sheet 23 in measurement, as shown in Fig. 5. The sensing portion 3 according to this embodiment is constituted of this sensor member 26 and the monochromatic light source 31, the lens 32, the lens 33 and the photoreceptor 34 of the aforementioned analytic unit 1.

The structure of an extraction cartridge set 200 storing the unused extraction cartridge 2 attached to the aforementioned blood glucose meter 100 is now described with reference to Figs. 3, 4, 6 and 8 to 11.

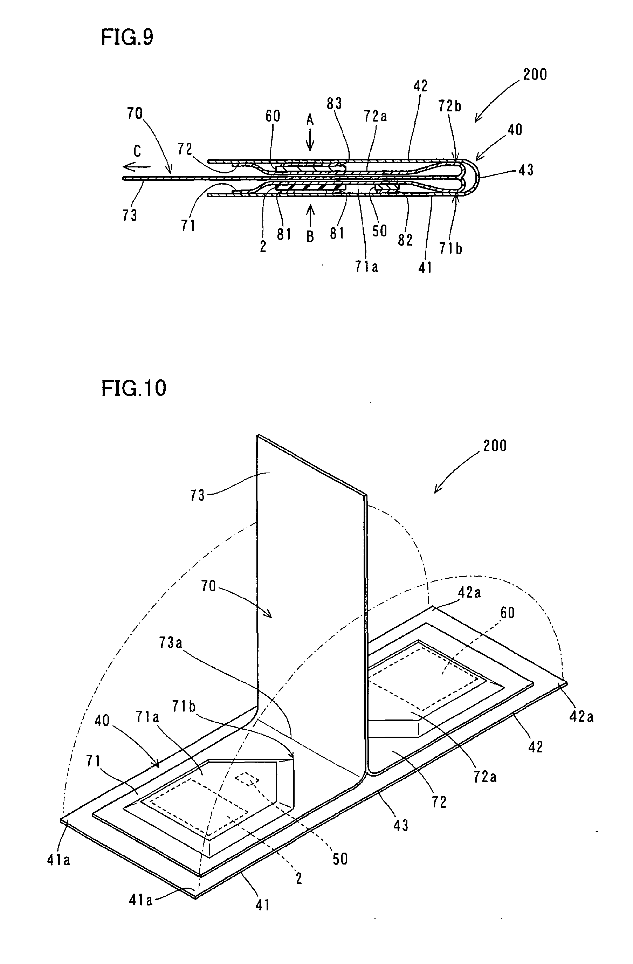

The extraction cartridge set 200 is so formed that the same stores the unused extraction cartridge 2 not yet attached to the blood glucose meter 100 in a dry state and can introduce a prescribed quantity of pure water into the mesh sheet 23 of the extraction cartridge 2 when attached to the blood glucose meter 100. The extraction cartridge set 200 according to this embodiment comprises a support member 40, the aforementioned extraction cartridge 2 (see Fig. 9), a desiccant 50 (see Fig. 9), a liquid supply member 60 (see Fig. 9) and a separative member 70, as shown in Figs. 8 and 9.

The support member 40 is provided in the form of a flexible sheet. This support member 40 has a three-layer structure obtained by stacking PET (polyethylene terephthalate), aluminum foil and polyethylene in this order. Further, the support member 40 is bent in a U-shaped manner, for directing the polyethylene layer inward. In addition, the support member 40 is constituted of a cartridge support portion 41, a liquid supply member support portion 42 opposed to the cartridge support portion 41 and a bent portion 43 coupling the cartridge support portion 41 and the liquid supply member support portion 42 with each other. Two corners 41a of the cartridge support portion 41 and two corners 42a of the liquid supply member support portion 42 are fixed to each other by thermally welding polyethylene layers constituting the surfaces of the corners 41a and 42a, as shown in Fig. 8. Thus, the support member 40 is formed in a substantially baggy shape, to be capable of holding (storing) the extraction cartridge 2 and the liquid supply member 60 in a region enclosed with the cartridge support portion 41, the liquid supply member support portion 42 and the bent portion 43. In the fixed state, the corners 41a and 42a of the cartridge support portion 41 and the liquid supply member support portion 42 are welded with easily cancelable welding force.

The extraction cartridge 2 is easily separably bonded to the inner side of the cartridge support portion 41 of the support member 40 with a pair of weakly adherent double-faced adhesive tapes 81, as shown in Fig. 9. Further, the extraction cartridge 2 is so arranged that the face of the mesh sheet 23 arranged on the skin is closer to the liquid supply member 60 (upward in Fig. 9).

The desiccant 50 is arranged on a position separated from the extraction cartridge 2 at a prescribed interval, and strongly fixed to the inner side of the cartridge support portion 41 of the support member 40 with an adhesive 82. The desiccant 50 is provided for inhibiting the mesh sheet 23 (see Fig. 6) of the extraction cartridge 2 from absorbing moisture etc. in the air and entering into a wet state.

The liquid supply member 60 is composed of absorbent cotton (cut cotton), and has a length of about 15 mm, a width of about 15 mm and a thickness of about 50 μm. Further, the liquid supply member 60 absorbs/holds pure water of a prescribed quantity (about 150 $\mu$l according to this embodiment). The pure water used in this embodiment has electric resistivity (specific resistance) of 18.3 M$\Omega$·cm, and is substantially an insulator (nonconductive substance). This liquid supply member 60 is strongly fixed to the inner side of the liquid supply member support portion 42 of the support member 40 with an adhesive 83 to be opposed to the mesh sheet 23 of the extraction cartridge 2 mounted on the cartridge support member 41 of the support member 40, as shown in Fig. 9. As hereinabove described, the liquid supply member 60 (about 15 mm by about 15 mm) having a larger area than the opening 28a (about 9 mm by about 3 mm) of the electrode sheet 28 arranged on the side of the measurement face 26a of the sensor member 26 of the extraction cartridge 2 is so employed that the pure water can be reliably supplied from the liquid supply member 60 to the mesh sheet 23 of the extraction cartridge 2 even if mutual positions slightly deviate from each other in contact between the extraction cartridge 2 and the liquid supply member 60. According to this embodiment, pure water (about 1.5 $\mu$l) corresponding to about 1 % of the pure water (about 150 $\mu$l) absorbed by the liquid supply member 60 is supplied from the liquid supply member 60 to the mesh sheet 23 of the extraction cartridge 2. Thus, the pure water absorbed/held by the liquid supply member 60 is so partially supplied to the mesh sheet 23 that a proper quantity of pure water can be supplied to the mesh sheet 23 even if the pure water absorbed/held by the liquid supply member 60 slightly evaporates, whereby the retention period of the extraction cartridge 2 can be lengthened.

The separative member 70 is provided in the form of a flexible sheet and has a three-layer structure consisting of a PET (polyethylene terephthalate) layer (inner surface), aluminum foil and a polyethylene layer (outer surface), similarly to the aforementioned support member 40. This separative member 70 is stored in the region enclosed with the cartridge support portion 41, the liquid supply member support portion 42 and the bent portion 43 of the U-shaped support member 40, as shown in Figs. 8 and 9. A prescribed part of the outer surface of the separative member 70 consisting of the polyethylene layer is mounted on a prescribed part of the inner surface of the support member 40 consisting of the polyethylene layer by thermal welding. The separative member 70 includes a cartridge storage portion 71 opposed to the cartridge support portion 41 of the support member 40, a liquid supply member storage portion 72 opposed to the liquid supply member support portion 42 of the support member 40 and a clip portion 73 held between the cartridge storage portion 71 and the liquid supply member storage portion 72, as shown in Fig. 9. The cartridge storage portion 71 and the liquid supply member storage portion 72 are integrally formed on the clip portion 73 to bisectionally extend from a prescribed edge 73a of the clip portion 73, as shown in Fig. 10.

The cartridge storage portion 71 has a recess portion 71a capable of storing the extraction cartridge 2 and the desiccant 50 between the same and the cartridge support portion 41 of the support member 40, as shown in Figs. 9, 10 and 11. This recess portion 71a of the cartridge storage portion 71 has a pentagonal home base shape in plan view. Further, the periphery of the recess portion 71a of the cartridge storage portion 71 is fixed to the inner side of the cartridge support portion 41 of the support member 40 by thermally welding the polyethylene layer constituting the outer surface to the polyethylene layer constituting the inner surface of the support member 40, as shown in Fig. 11. Thus, the extraction cartridge 2 and the desiccant 50 can be held in a space, sealed against the exterior, formed by the cartridge support portion 41 of the support member 40 and the recess portion 71a of the cartridge storage portion 71 of the separative member 70. Consequently, the extraction cartridge 2 can be preserved in a space formed by the support member 40 and the separative member 70 in a dry state. Further, the recess portion 71a of the cartridge storage portion 71 is so formed in the home base shape that the separative member 70 is stripped from the support member 40 through a starting point formed by a corner 71b of the recess portion 71a of the cartridge storage portion 71 dissimilarly to a case of forming the recess portion 71a in a square shape, whereby the separative member 70 can be easily detached from the support member 40 with small force.

The liquid supply member storage portion 72 has a recess portion 72a capable of storing the liquid supply member 60 in the state containing pure water between the same and the liquid supply member support portion 42 of the support member 40. This recess portion 72a of the liquid supply member storage portion 72 has a pentagonal home base shape in plan view. Further, the periphery of the recess portion 72a of the liquid supply member storage portion 72 is fixed to the inner side of the liquid supply member support portion 42 of the support member 40 by thermally welding the polyethylene layer constituting the outer surface to the polyethylene layer constituting the inner surface of the support member 40. Thus, the liquid supply member 60 containing pure water can be held in a space, sealed against the exterior, formed by the liquid supply member support portion 42 of the support member 40 and the recess portion 72a of the liquid supply member storage portion 72 of the separative member 70. Further, the recess portion 72a of the liquid supply member storage portion 72 is so formed in the home base shape that the separative member 70 is stripped from the support member 40 through a starting point formed by a corner 72b of the recess portion 72a of the liquid supply member storage portion 72 dissimilarly to a case of forming the recess portion 72a in a square shape, whereby the separative member 70 can be easily detached from the support member 40 with small force.

The clip portion 73 is provided to be clipped by the subject when detaching the separative member 70 from the support member 40. This clip portion 73 protrudes toward the exterior from a position opposite to the bent portion 43 of the support member 40 by a prescribed length, as shown in Fig. 9. The subject clips this protruding part of the clip portion 73 and pulls the same along arrow C so that this tensile force is transmitted to the cartridge storage portion 71 and the liquid supply member storage portion 72 through the clip portion 73, whereby the cartridge storage portion 71 and the liquid supply member storage portion 72 can be gradually stripped from the cartridge support portion 41 and the liquid supply member support portion 42 of the support member 40 through starting points formed by the corners 71b and 72b of the respective recess portions 71a and 72a.

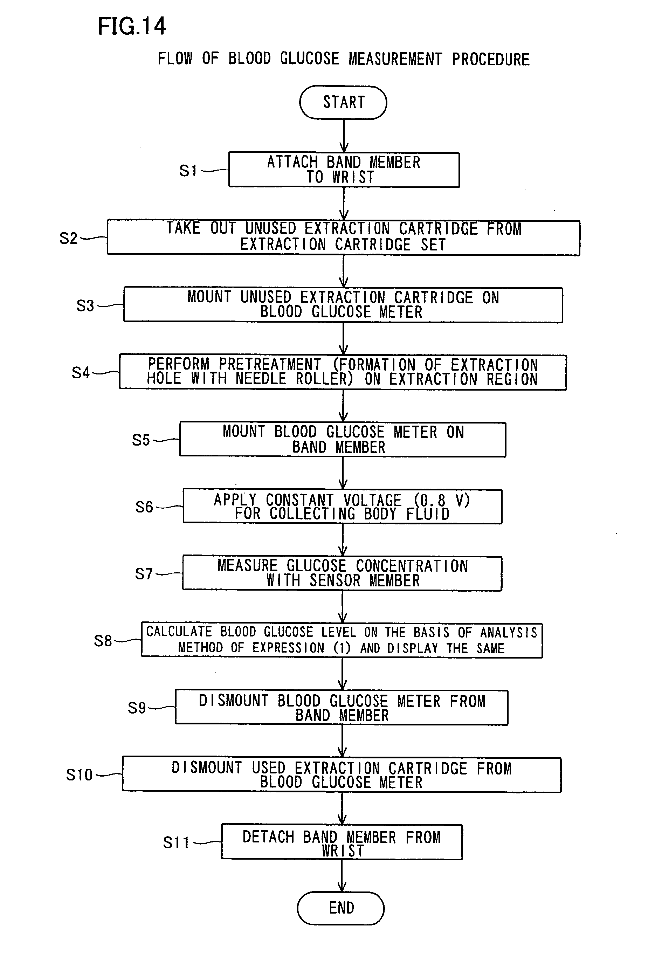

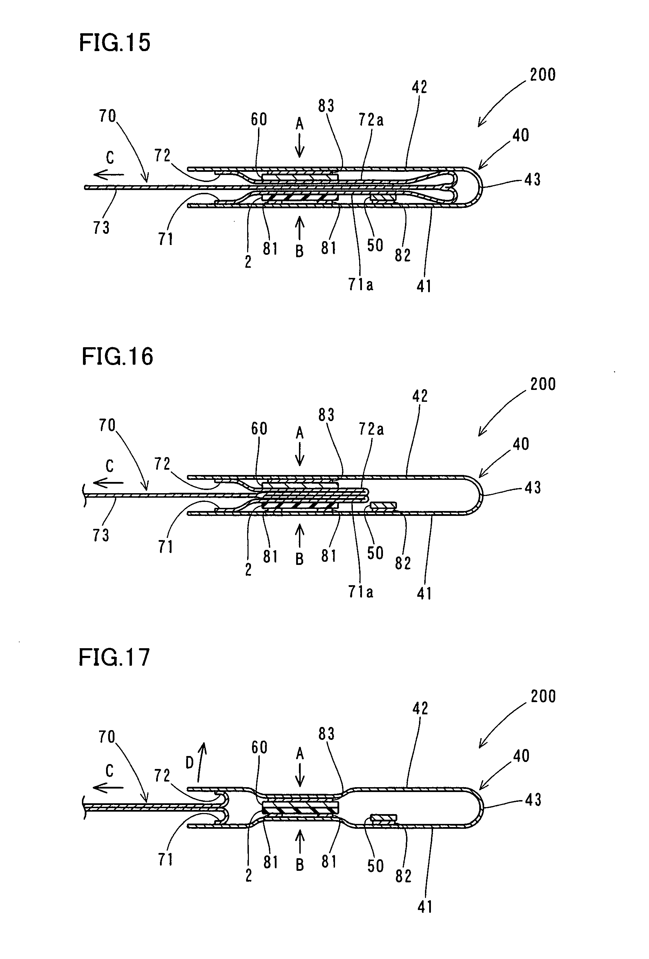

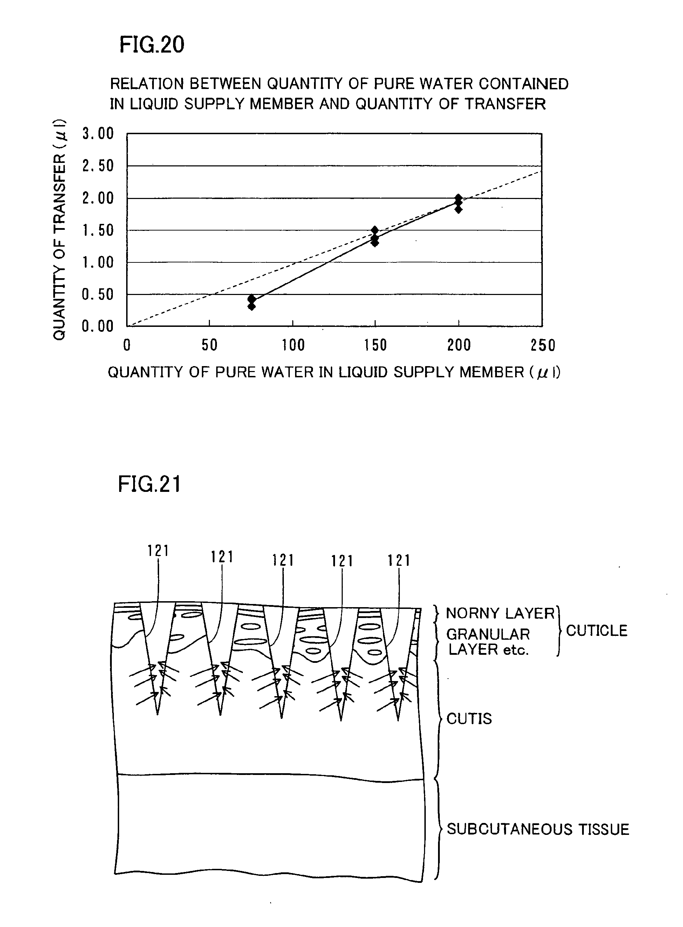

Fig. 14 is a flow chart showing an operation procedure of blood glucose measurement with the blood glucose meter shown in Fig. 1. Figs. 15 to 19 are diagrams for illustrating an operation at a time of taking out the extraction cartridge from the extraction cartridge set shown in Fig. 8. Fig. 20 is a correlation diagram showing the relation between the quantities of pure water absorbed/held in liquid supply members of the extraction cartridge set and the quantities of pure water transferred (supplied) from the liquid supply members to mesh sheets of the extraction cartridge. Figs. 21 to 24 are model diagrams for illustrating the principle of glucose extraction with the blood glucose meter shown in Fig. 1. The operation procedure of the blood glucose measurement with the blood glucose meter 100 is now described with reference to Figs. 1, 5, 6, 9 and 12 to 24.

First, the band member 110 is attached to the wrist 120 of the subject at a step S1 shown in Fig. 14. At this time, the band member 110 is so attached that the measurement portion (extraction region) is positioned in the opening (not shown) of the fixture of the band member 110.

At a step S2, the subject takes out the unused extraction cartridge 2 from the extraction cartridge set 200. More specifically, the subject holds the extraction cartridge set 200 by clipping the outer sides of the cartridge support portion 41 and the liquid supply member 42 of the support member 40 of the extraction cartridge set 200 shown in Figs. 8 and 9 with two fingers from the directions along arrows A and B. This extraction cartridge set 200 stores the unused dry extraction cartridge 2 and the liquid supply member 60 absorbing/holding the pure water of the prescribed quantity (about 150 $\mu$l) in a state not in contact with each other. Then, the subject clips the clip portion 73 of the separative member 70 outwardly protruding from the support member 40 of the extraction cartridge set 200 with his/her fingers and pulls the same along arrow C. Thus, the clip portion 73 of the separative member 70 is moved along arrow C, as shown in Figs. 15 and 16. Following this movement of the clip portion 73 along arrow C, the cartridge storage portion 71 and the liquid supply member storage portion 72 of the separative member 70 are gradually stripped from the cartridge support portion 41 and the liquid supply member support portion 42 of the support member 40 through the starting points formed by the corners 71b and 72b of the recess portions 71a and 72a of the cartridge storage portion 71 and the liquid supply member storage portion 72.

As shown in Fig. 17, the separative member 70 is removed from between the dry extraction cartridge 2 and the liquid supply member 60 absorbing/holding the pure water, while the cartridge support portion 41 and the liquid supply member support portion 42 of the support member 40 are pressed by the fingers of the subject from the directions along arrows A and B. Thus, the mesh sheet 23 of the dry extraction cartridge 2 and the liquid supply member 60 absorbing/holding the pure water come into contact with each other, and about 1% (about 1.5 $\mu$l) of the pure water (about 150 $\mu$l) absorbed/held in the liquid supply member 60 is transferred (supplied) to the mesh sheet 23 (see Fig. 6) of the extraction cartridge 2.

An experiment conducted as to transfer accuracy (transfer rate) and reproducibility in the case of employing the transfer (supply) method according to this embodiment is described with reference to Fig. 20. Referring to Fig. 20, the quantities ($\mu$l) of pure water absorbed/held in liquid supply members 60 of cut cotton are taken on the axis of abscissas, and the quantities ($\mu$l) of pure water transferred from the liquid supply members 60 to mesh sheets 23 of nylon are taken on the axis of ordinates. In this experiment, pure water was transferred from the liquid supply members 60 to the mesh sheets 23 by bringing three types of liquid supply members 60 absorbing/holding 75 $\mu$l of pure water, 150 $\mu$l of pure water and 200 $\mu$l of pure water respectively into contact with the mesh sheets 23 three times respectively. Then, the quantities of pure water transferred from the liquid supply members 60 to the mesh sheets 23 were measured respectively. As a result, the average of the quantity of pure water transferred from the liquid supply member 60 to the mesh sheet 23 was about 0.40 $\mu$l when the quantity of pure water absorbed/held in the liquid supply member 60 was 75 $\mu$l, as shown in Fig. 20. When the quantity of pure water absorbed/held in the liquid supply member 60 was 150 $\mu$l, the average of the quantity of pure water transferred from the liquid supply member 60 to the mesh sheet 23 was about 1.40 $\mu$l. When the quantity of pure water absorbed/held in the liquid supply member 60 was 200 $\mu$l, the average of the quantity of pure water transferred from the liquid supply member 60 to the mesh sheet 23 was about 1.90 $\mu$l.

Thus, it has been proved that the average (about 0.40 $\mu$l) of the quantity of pure water transferred from the liquid supply member 60 to the mesh sheet 23 is about 0.5 % of the quantity (75 $\mu$l) of pure water absorbed/held in the liquid supply member 60 when the quantity of pure water absorbed/held in the liquid supply member 60 is 75 $\mu$l. Further, it has been proved that the average (about 1.40 $\mu$l) of the quantity of pure water transferred from the liquid supply member 60 to the mesh sheet 23 is about 1.0 % of the quantity (150 $\mu$l) of pure

water absorbed/held in the liquid supply member 60 when the quantity of pure water absorbed/held in the liquid supply member 60 is 150 $\mu$l. In addition, it has been proved that the average (about 1.90 $\mu$l) of the quantity of pure water transferred from the liquid supply member 60 to the mesh sheet 23 is about 1.0 % of the quantity (200 $\mu$l) of pure water absorbed/held in the liquid supply member 60 when the quantity of pure water absorbed/held in the liquid supply member 60 is 200 $\mu$l. Consequently, it has been recognized that the transfer rate of pure water from the liquid supply member 60 to the mesh sheet 23 (proportion of the transfer rate with respect to the quantity of pure water contained in the liquid supply member 60) enlarges (transfer rate of about 1.0 %) when the quantity of pure water absorbed/held in the liquid supply member 60 is large (150 $\mu$l and 200 $\mu$l) as compared with the case where the quantity of pure water absorbed/held in the liquid supply member 60 is small (75 $\mu$l) (transfer rate of about 0.5 %). This is conceivably because the pure water hardly outwardly moves from the liquid supply member 60 if the quantity of the pure water absorbed/held in the liquid supply member 60 is small.

Further, it has been recognized that the transfer rates of pure water from the liquid supply members 60 to the mesh sheets 23 hardly differ from each other in the cases where the quantities of pure water absorbed/held in the liquid supply members 60 are 150 $\mu$l and 200 $\mu$l. Thus, it has been recognized that the transfer rate of pure water from the liquid supply member 60 of cut cotton (absorbent cotton) to the mesh sheet 23 of nylon tends to stabilize at about 1.0 % when the quantity of pure water absorbed/held in the liquid supply member 60 is relatively large (about 150 $\mu$l). A CV (coefficient of variation: scale for evaluating substantial data dispersion) value as reproducibility of the transfer rate was about 10 %. Thus, it has been proved that reproducibility of transfer of pure water from the liquid supply member 60 of cut cotton (absorbent cotton) to the mesh sheet 23 of nylon is also sufficiently high.

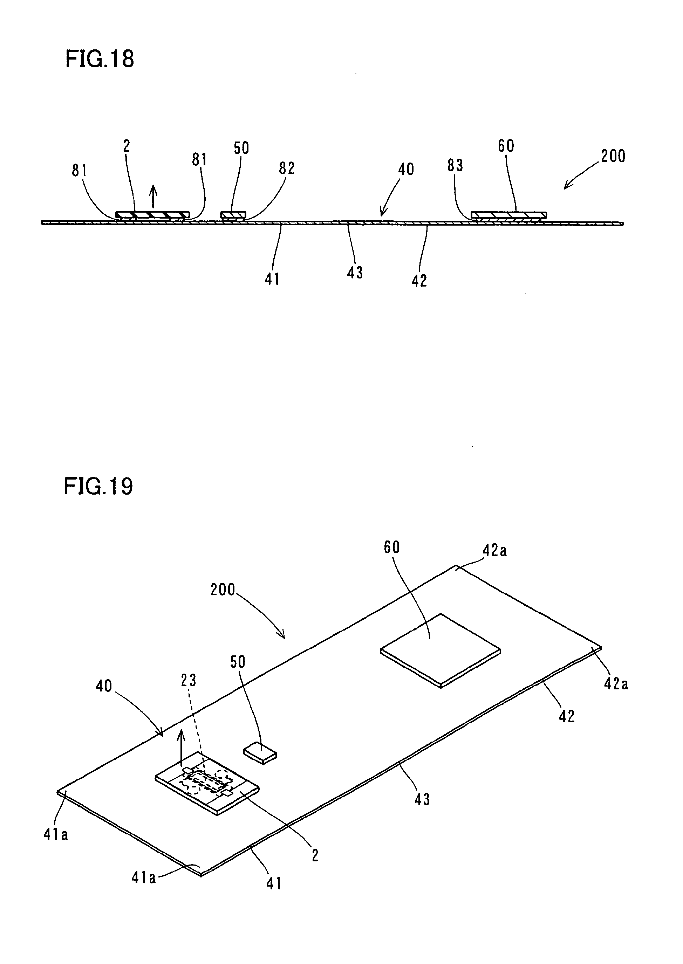

After the pure water is transferred from the liquid supply member 60 to the mesh sheet 23 as described above, the two corners 41a of the cartridge support portion 41 of the support member 40 and the two corners 42a of the liquid supply member support portion 42 are released from the fixed state and the liquid supply member support portion 42 of the support member 40 is rotated about the bent portion 43 along arrow D shown in Fig. 17, thereby attaining the state shown in Figs. 18 and 19. Then, the extraction cartridge 2 including the mesh sheet 23 containing the pure water of a prescribed quantity (about 1.5 $\mu$l) is removed from the pair of double-faced adhesive tapes 81 whose single surfaces are mounted on the cartridge support member 41.

At a step S3, the unused extraction cartridge 2 including the mesh sheet 23 containing the pure water of the prescribed quantity (about 1.5 $\mu$l) is mounted on the blood glucose meter 100 by engaging the pair of engaging hooks 15 of the blood glucose meter 100 with the pair of mounting holes 21 of the extraction cartridge 2. At a step S4, pretreatment is performed by forming the plurality of minute extraction holes 121 in the skin of the extraction region with the needle roller 130 (see Fig. 12), as shown in Fig. 13. Body fluid containing glucose, originally residing in the cutis of the skin, gradually oozes out into the extraction holes 121 formed in the skin through the pretreatment with this needle roller 130, as shown in Fig. 21. At a step S5, the blood glucose meter 100 is mounted on the fixture of the band member 110. Thus, the blood glucose meter 100 is attached to the wrist 120 of the subject while the lower side of the extraction cartridge 2 is in contact with the skin of the subject. More specifically, the measurement portion (extraction region) of the wrist 120 swells through fastening of the band member 110 (see Fig. 1) so that the mesh sheet 23 and the skin come into contact with each other and the central portion of the mesh sheet 23 moves upward to come into contact with the lower surface (measurement face 26a) of the sensor member 26, as shown in Fig. 5.

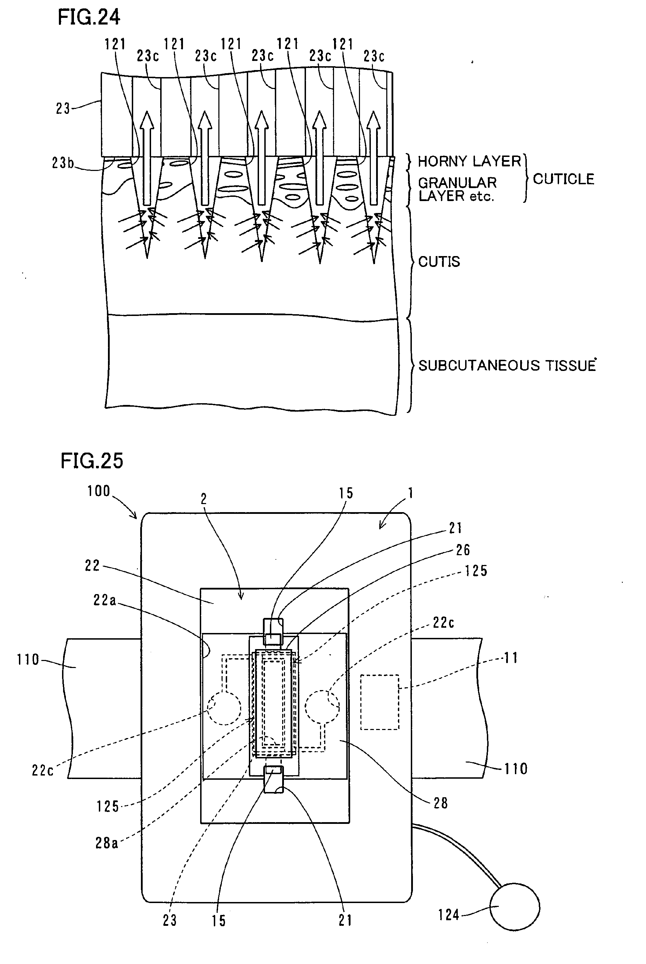

At this time, the pure water contained in the mesh sheet 23 penetrates into the extraction holes 121 formed in the skin, as shown in Fig. 22. As shown in Fig. 23, the body fluid oozing out into the extraction holes 121 of the skin and the pure water from the mesh sheet 23 intermingle with each other, so that the body fluid in the extraction holes 121 is diffused into the pure water held in the through-holes 23c of the mesh sheet 23. Thus, the osmotic pressure in the extraction holes 121 becomes lower than the osmotic pressure in the cutis of the skin, whereby the body fluid oozes out from the cutis into the extraction holes 121 again. Consequently, the body fluid oozing out through the extraction holes 121 formed in the skin is diffused into the pure water held in the through-holes 23c of the mesh sheet 23 to some extent, before the voltage is applied from the constant-voltage power source 13 to the anode 24 and the cathode 25 of the extraction cartridge 2. In this state, the constant voltage (0.8 V) is applied from the constant-voltage power source 13 to the anode 24 and the cathode 25 for about 3 minutes by pressing a measurement starting switch (not shown) of the blood glucose meter 100 at a step S6 in Fig. 14. Thus, charged ionic components present in the extraction holes 121 positively move toward the anode 24 and the cathode 25, whereby the body fluid containing glucose of a quantity detectable by the sensing portion 3 is collected from the organism into the pure water held in the mesh sheet 23, as shown in Fig. 24.

The mechanism of collection of the body fluid into the pure water held in the mesh sheet 23 through application of the voltage to the anode 24 and the cathode 25 of the extraction cartridge 2 is now described. When the voltage is applied from the constant-voltage power source 13 to the anode 24 and the cathode 25, the collector 24a of the anode 24 is positively (+) charged, while the collector 25a of the cathode 25 is negatively (-) charged. The activated carbon electrodes 24b and 25b stuck to the lower portions of the collectors 24a and 25a of the anode 24 and the cathode 25 respectively have polarity. Therefore, a lower portion in the activated carbon electrode 24b closer to the anode 24 is positively (+) charged, while a lower portion in the activated carbon electrode 25b closer to the cathode 25 is negatively (-) charged. Thus, sodium ions ($Na^+$) and chloride ions ($Cl^-$) contained in the body fluid extracted into the extraction holes 121 move toward the activated carbon electrode 25b and the activated carbon electrode 24b respectively in the pure water held in the mesh sheet 23. Following this movement of the sodium ions ($Na^+$) and the chloride ions ($Cl^-$) in the body fluid toward the activated carbon electrodes 25b and 24b, biochemical components such as glucose in the body fluid move into the pure water held in the mesh sheet 23. Then, the biochemical components such as glucose reach the measurement face 26a of the sensor member 26.

In parallel with the step S6, the quantity of glucose is measured with the sensor member 26 at a step S7. This calculation of the quantity of glucose at the step S7 is continuously carried out every prescribed time (every second, for example) while the constant-voltage power source 13 applies the constant voltage to the anode 24 and the cathode 25.

More specifically, glucose oxidase (GOD) which is the oxidase in the mixed gel applied to the measurement face 26a of the sensor member 26 according to this embodiment so acts as the catalyst that hydrogen peroxide ($H_2O_2$) and gluconic acid are formed from the glucose reaching the measurement face 26a. Then, peroxidase (POD) which is the oxidoreductase in the mixed gel applied to the measurement face 26a of the sensor member 26 so acts as the catalyst that active oxygen ($O^*$) and water ($H_2O$) are formed from hydrogen peroxide ($H_2O_2$). Further, the chromogenic dye in the mixed gel applied to the measurement face 26a of the sensor member 26 pigments by reacting with the formed active oxygen ($O^*$).

Thus, light passing through the second light guide layer 26d (see Fig. 7) of the sensor member 26 in contact with the mesh sheet 23 containing the pure water with total reflection is absorbed by the chromogenic dye pigmenting in response to the quantity of glucose extracted from the organism, and thereafter reaches the photoreceptor 34. The control portion 11 calculates the quantity of glucose on the basis of the signal output from this photoreceptor 34. As the chromogenic dye contained in the pure water, N,N-bis(2-hydroxy-3-sulfopropyl)tolidine dipotassium salt, 3,3',5,5'-tetramethyl benzylidene or the like can be employed, for example. At a step S8, the control portion 11 calculates the blood glucose level from the quantity of glucose calculated every second at the step S7 on the basis of an analysis method of the following expression (1) and displays the calculated glucose extraction and blood glucose level per unit time on the display portion 12 (see Fig. 1).

$$BG = C/P$$
$$= C/(A \times I + B) \dots (1)$$

In the above expression (1), "BG" represents the calculated blood glucose level, "C" represents the quantity of the glucose extraction per unit time calculated from the quantity of glucose per second obtained at the step S7, "P" represents glucose permeability (glucose passableness) of the extraction region, and "I" represents the average current value measured by the ammeter 14 during application of the voltage. "A" and "B" are constants previously decided by experiments. At a step S9 in Fig. 14, the blood glucose meter 100 is dismounted from the fixture of the band member 110.

At a step S10, the used extraction cartridge 2 is dismounted from the blood glucose meter 100 by releasing the pair of mounting holes 21 of the extraction cartridge 2 and the pair of engaging hooks 15 of the blood glucose meter 100 from the engaging state. This used extraction cartridge 2 may be thrown away, or may be subjected to proper treatment and recycled. Thereafter the band member 110 is detached from the wrist 120 of the subject at a step S11. Thus, the blood glucose measurement with the blood glucose meter 100 is ended.

According to this embodiment, as hereinabove described, the mesh sheet 23 of nylon capable of holding the pure water for supplying glucose from the skin contact face 23b to the measurement face 26a is provided and the mesh sheet 23 is so formed as to keep the distance between the measurement face 26a and the skin substantially constant while the same is in contact with the skin, whereby the distance between the measurement face 26a and the skin at the time of extracting glucose can be kept substantially constant. Therefore, the distance between the measurement face 26a and the skin substantially remains unchanged, and the time up to arrival of the glucose at the sensor member 26 can be kept substantially constant. Thus, occurrence of a measurement error resulting from dispersion in the distance between the skin and the sensor member 26 can be suppressed although extraction and detection of the glucose are performed in parallel with each other.

According to this embodiment, as hereinabove described, the mesh sheet 23 is formed to remain dry during preservation of the extraction cartridge 2, whereby the enzymes (glucose oxidase (GOD) and peroxidase (POD)) contained in the sensor member 26 can be inhibited from deteriorating in a short time due to contact with moisture.

According to this embodiment, as hereinabove described, the mesh sheet 23 having the plurality of trough-holes 23c is so employed that the pure water supplied from the liquid supply member 60 can be easily held in the plurality of through-holes 23c constituting the mesh of the mesh sheet 23, whereby the glucose extracted from the subject can be easily made to reach the measurement face 26a through the pure water in the through-holes 23c.

According to this embodiment, as hereinabove described, the single extraction cartridge 2 is so formed that the anode 24 and the cathode 25 connected to the constant-voltage power source 13 of the blood glucose meter 100 come into contact with the single mesh sheet 23, whereby an electric field can be supplied to the skin of the wrist 120 of the subject by the anode 24 and the cathode 25 connected to the constant-voltage power source 13 through the mesh sheet 23, so that the glucose extracted from the skin of the subject can be made to move toward the anode 24 and the cathode 25. Thus, the glucose moving toward the anode 24 and the cathode 25 is held in the pure water held in the single mesh sheet 23, whereby the glucose can be more efficiently extracted from the skin of the subject.

The embodiment disclosed this time must be considered as illustrative in all points and not restrictive. The range of the present invention is shown not by the above description of the embodiment but by the scope of claim for patent, and all modifications within the meaning and range equivalent to the scope of claim for patent are included.

For example, while the above embodiment has shown the example of employing the mesh sheet of nylon having a network structure as an analyte holding member according to the present invention, the present invention is not restricted to this but a mesh sheet formed by an insulating material such as paper or resin other than nylon may be employed. Further, a member of a porous structure formed by perforating resin or the like with a laser or the like may be employed in place of the mesh sheet.

While the above embodiment has shown the example of employing the pure water which is a substantially nonconductive substance as the liquid supplied from the liquid supply member to the mesh sheet, the present invention is not restricted to this but another conductive substance such as a physiological salt solution other than the pure water may be employed as the liquid supplied from the liquid supply member to the mesh sheet.

While an optical sensor has been employed as the sensor member sensing glucose in the aforementioned embodiment, the present invention is not restricted to this but an electric sensor such as a sensor electrode assembly described in International Patent Laying-Open No. WO96/00110 as the sensor member sensing glucose. In this case, a sensor electrode may be so arranged to come into contact with the sensor contact face 23a of the mesh sheet 23 in place of the sensor member 26 shown in Fig. 5, a current value output portion outputting a current value detected by the sensor electrode to the control portion 11 (see Fig. 3) may be provided, and glucose oxidase (GOD) may be held in the mesh sheet 23 or the sensor electrode. According to this structure, hydrogen peroxide is formed when glucose extracted from the subject into the pure water in the mesh sheet 23 reacts through catalytic action of GOD, and electric charge resulting from this formation of hydrogen peroxide is supplied to the sensor electrode thereby generating a current. The current value output portion outputs the current value of the generated current to the control portion 11, and the control portion 11 calculates the quantity of glucose on the basis of this current value.

While the above embodiment has shown the example of applying the voltage to the electrodes of the extraction cartridge with the constant-voltage power source, the present invention is not restricted to this but a constant-current power source may be employed in place of the constant-voltage power source, or glucose may be extracted from the organism by natural extraction, supersonic extraction, extraction with a negative pressure or the like without employing the constant-voltage power source.

While the above embodiment has shown the example of storing the extraction cartridge and the liquid supply member (absorbent cotton) in the same support member, the present invention is not restricted to this but a support member storing the extraction cartridge and another support member storing the liquid supply member (absorbent cotton) may be individually provided.

While the above embodiment has shown the example of storing the extraction cartridge and the liquid supply member (absorbent cotton) in the flexible sheetlike support member, the present invention is not restricted to this but the extraction cartridge and the liquid supply member (absorbent cotton) may be stored in a nonflexible boxlike member.

While the above embodiment has shown the example of employing the flexible sheetlike separative member, the present invention is not restricted to this but a nonflexible platelike member or the like may be employed as the separative member. Further, the separative member may not be rendered separable from the support member.

While the above embodiment has described the example of separating the separative member from the support member by clipping and pulling the clip portion of the separative member, the present invention is not restricted to this but the separative member may be separated from the support member by forming a hole capable of receiving a finger of the subject in a portion of the separative member protruding from the support member, inserting the finger into the hole of the separative member and pulling the separative member. Alternatively, a ringlike member for pulling the separative member by inserting the finger thereinto may be separately mounted on the separative member. According to this structure, the separative member can be dismounted from the support member with one finger.

While the above embodiment has shown the example of forming the recess portions of the cartridge storage portion and the liquid supply member storage portion in the linear home base shapes so that the separative member is separated from the support member through the starting points formed by the corners of the recess portions of the respective ones of the cartridge storage portion and the liquid supply member storage portion, the present invention is not restricted to this but parts of the recess portions of the cartridge storage portion and the liquid supply member storage portion forming the starting points for separating the separative member from the support member may be arcuately formed. Alternatively, the recess portions of the cartridge storage portion and the liquid supply member storage portion may be circularly or quadrangularly formed.

While the above embodiment has shown the example of forming the extraction cartridge 2 to have the anode 24 connected to the anodic side of the constant-voltage power source 13 of the blood glucose meter 100 and the cathode 25 connected to the cathodic side of the constant-voltage power source 13 of the blood glucose meter 100 as shown in Fig. 2, the present invention is not restricted to this but the extraction cartridge 2 may be formed to have two cathodes 125 connected to the cathodic side of the constant-voltage power source 13 of the blood glucose meter 100 as in a modification shown in Fig. 25. At this time, an anodic terminal 124 electrically connected to the anodic side of the constant-voltage power source 13 of the blood glucose meter 100 is separately provided while this anodic terminal portion 124 is set on another region of the skin of the subject. In this case, a conductive liquid such as a physiological salt solution is preferably used in place of the pure water.

While the above embodiment has shown the example of employing absorbent cotton (cut cotton) which is a kind of nonwoven fabric as the liquid supply member, the present invention is not restricted to this but a liquid supply member composed of nonwoven fabric other than absorbent cotton (cut cotton) or a liquid supply member composed of a material other than nonwoven fabric may be employed so far as the material can correctly control the quantity of the supplied liquid by coming into contact with the mesh sheet (liquid holding member), and sponge, paper, filter paper, gel or the like may be employed, for example.

While the above embodiment has described the example of applying the voltage to the anode and the cathode with the DC-system constant-voltage power source, the present invention is not restricted to this but the voltage may be applied to the anode and the cathode with an AC-system constant-voltage power source.

While the above embodiment has described the example of applying the present invention to the blood glucose meter extracting glucose from an organism and calculating the blood glucose level, the present invention is not restricted to this but the present invention may be applied to an analyte extractor extracting another analyte other than glucose from an organism. As the analyte extracted by the analyte extractor to which the present invention is applicable, a biochemical component or a medicine administered to the subject, for example. As the biochemical component, albumin, globulin or an enzyme which is protein regarded as a kind of biochemical component can be listed. As a biochemical component other than protein, creatinine, creatine, uric acid, amino acid, fructose, galactose, pentose, glycogen, lactic acid, pyruvic acid or a ketone body can be listed. As the medicine, a digitalis preparation, theophylline, an arrhythmia preparation, an antiepileptic, an amino acid glycoside antibiotic, a glycopeptide-based antibiotic, an antithrombotic agent or an immunosuppressive agent can be listed.

In the blood glucose meter according to the aforementioned embodiment or in the case of applying the present invention to the analyte extractor extracting another analyte other than glucose from an organism, the sensing portion and the control portion may be so formed as to analyze protein or another biochemical component other than protein and a medicine with another measuring method such as HPLC (High Performance Liquid Chromatography).

## Claims

1. An analyzer (100) for analyzing an analyte extracted from a subject, comprising:

   a sensor member (26) including a supplied face (26a) supplied with said analyte extracted from said subject or a product resulting from reaction of said analyte;
   signal output means outputting a signal based on said analyte or said product supplied to said supplied face; and
   a liquid holding member (23) provided with a first face (23a) coming into contact with said supplied face and a second face (23b) coming into contact with the skin of said subject and capable of holding a liquid for supplying said analyte or said product from said second face to said supplied face, wherein
   said liquid holding member is so formed as to keep the distance between said supplied face and said skin substantially constant while the same is in contact with said skin.

2. The analyzer according to claim 1, wherein said signal output means includes:

   a light source (31) applying light to said sensor member, and
   a photodetector (34) detecting the light passing through said sensor member.

3. The analyzer according to claim 1 or 2, wherein said signal output means outputs the signal based on said analyte or said product in parallel with the supply of said analyte or said product to said supplied face.

**4.** The analyzer according to any of claims 1 to 3, further comprising:

 an electrode (24, 25) so arranged as to come into contact with the first face of said liquid holding member, and
 a power source (13) connected to said electrode.

**5.** The analyzer according to any of claims 1 to 4, wherein
said liquid holding member is provided in the form of a sheet.

**6.** The analyzer according to any of claims 1 to 5, wherein
said liquid holding member keeps a substantially constant thickness when held between said supplied face and said skin.

**7.** The analyzer according to any of claims 1 to 6, wherein
said liquid holding member is made of nylon.

**8.** The analyzer according to any of claims 1 to 7, wherein
said liquid holding member has a thickness of not more than 1 mm.

**9.** The analyzer according to any of claims 1 to 8, wherein
said liquid holding member includes a plurality of through-holes (23c) passing through said first face and said second face.

**10.** A cartridge (2) for extracting an analyte attachable to an analyzer (100) analyzing an analyte extracted from a subject, comprising:

 a sensor member (26) including a supplied face (26a) supplied with said analyte extracted from said subject or a product resulting from reaction of said analyte; and
 a liquid holding member (23) provided with a first face (23a) coming into contact with said supplied face and a second face (23b) coming into contact with the skin of said subject and capable of holding a liquid for supplying said analyte or said product from said second face to said supplied face, wherein
 said liquid holding member is so formed as to keep the distance between said supplied face and said skin substantially constant while the same is in contact with said skin.

**11.** The cartridge for extracting an analyte according to claim 10, wherein
said sensor member contains a first enzyme serving as a catalyst for said analyte in said supplied face.

**12.** The cartridge for extracting an analyte according to claim 11, wherein
said sensor member further contains a second enzyme serving as a catalyst for a first substance produced from said analyte in said supplied face.

**13.** The cartridge for extracting an analyte according to claim 12, wherein
said sensor member further contains a chromogenic dye reacting with a second substance produced from said first substance.

**14.** The cartridge for extracting an analyte according to any of claims 10 to 13, wherein
said liquid holding member is provided in the form of a sheet.

**15.** The cartridge for extracting an analyte according to any of claims 10 to 14, wherein
said liquid holding member keeps a substantially constant thickness when held between said supplied face and said skin.

**16.** The cartridge for extracting an analyte according to any of claims 10 to 15, wherein
said liquid holding member is made of nylon.

**17.** The cartridge for extracting an analyte according to any of claims 10 to 16, wherein
said liquid holding member has a thickness of not more than 1 mm.

**18.** The cartridge for extracting an analyte according to any of claims 10 to 17, wherein
said liquid holding member includes a plurality of through-holes (23c) passing through said first face and said second face.

**19.** The cartridge for extracting an analyte according to claim 18, wherein
the plurality of through-holes of said liquid holding member are arranged in the form of a lattice.

**20.** The cartridge for extracting an analyte according to any of claims 10 to 19, further comprising an electrode (24, 25) arranged to be held between said sensor member and said liquid holding member.

**21.** The cartridge for extracting an analyte according to any of claims 10 to 20, wherein
said liquid holding member is so arranged as to cover the whole of said supplied face.

FIG.1

FIG.2

## FIG.3

## FIG.4

FIG.5

FIG.6

FIG.7

FIG.8

FIG.9

FIG.10

FIG.11

FIG.12

FIG.13

121  121  121  121  121

HORNY LAYER
GRANULAR
LAYER etc.    } CUTICLE

CUTIS

SUBCUTANEOUS TISSUE

# FIG.14

FLOW OF BLOOD GLUCOSE MEASUREMENT PROCEDURE

```
                    ( START )
                        |
                        v
         S1 —— | ATTACH BAND MEMBER
                 |      TO WRIST        |
                        |
                        v
    S2 —— | TAKE OUT UNUSED EXTRACTION CARTRIDGE FROM
           |         EXTRACTION CARTRIDGE SET          |
                        |
                        v
     S3 —— | MOUNT UNUSED EXTRACTION CARTRIDGE ON
             |       BLOOD GLUCOSE METER            |
                        |
                        v
    S4 —— | PERFORM PRETREATMENT (FORMATION OF EXTRACTION
           | HOLE WITH NEEDLE ROLLER) ON EXTRACTION REGION  |
                        |
                        v
      S5 —— | MOUNT BLOOD GLUCOSE METER ON
              |        BAND MEMBER          |
                        |
                        v
      S6 —— | APPLY CONSTANT VOLTAGE (0.8 V)
              |  FOR COLLECTING BODY FLUID    |
                        |
                        v
      S7 —— | MEASURE GLUCOSE CONCENTRATION
              |      WITH SENSOR MEMBER        |
                        |
                        v
    S8 —— | CALCULATE BLOOD GLUCOSE LEVEL ON THE BASIS OF ANALYSIS
           | METHOD OF EXPRESSION (1) AND DISPLAY THE SAME       |
                        |
                        v
      S9 —— | DISMOUNT BLOOD GLUCOSE METER FROM
              |         BAND MEMBER             |
                        |
                        v
   S10 —— | DISMOUNT USED EXTRACTION CARTRIDGE FROM
           |         BLOOD GLUCOSE METER           |
                        |
                        v
      S11 —— | DETACH BAND MEMBER FROM
               |        WRIST          |
                        |
                        v
                    (  END  )
```

FIG.15

FIG.16

FIG.17

## FIG.18

## FIG.19

## FIG.20

RELATION BETWEEN QUANTITY OF PURE WATER CONTAINED
IN LIQUID SUPPLY MEMBER AND QUANTITY OF TRANSFER

QUANTITY OF PURE WATER IN LIQUID SUPPLY MEMBER ($\mu$l)

## FIG.21

FIG.22

121   121   121   121   121
23c   23c   23c   23c   23c

23

23b

HORNY LAYER
GRANULAR
LAYER etc.
}CUTICLE

}CUTIS

}SUBCUTANEOUS TISSUE

FIG.23

121   121   121   121   121
23c   23c   23c   23c   23c

23

23b

HORNY LAYER
GRANULAR
LAYER etc.
}CUTICLE

}CUTIS

}SUBCUTANEOUS TISSUE

## FIG.24

HORNY LAYER
GRANULAR LAYER etc. } CUTICLE

CUTIS

SUBCUTANEOUS TISSUE

## FIG.25

<table>
<tr><td colspan="2" align="center">**INTERNATIONAL SEARCH REPORT**</td><td>International application No.<br>PCT/JP2006/300121</td></tr>
</table>

| A. CLASSIFICATION OF SUBJECT MATTER |
|---|
| **A61B5/1459**(2006.01), **A61B5/15**(2006.01), **A61B5/151**(2006.01), **A61N1/30** (2006.01), **G01N33/50**(2006.01), *G01N33/66*(2006.01) |
| According to International Patent Classification (IPC) or to both national classification and IPC |

| B. FIELDS SEARCHED |
|---|
| Minimum documentation searched (classification system followed by classification symbols)<br>**A61B5/1459**(2006.01), **A61B5/15**(2006.01), **A61B5/151**(2006.01), **A61N1/30** (2006.01), **G01N33/50**(2006.01), *G01N33/66*(2006.01) |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
   JMEDPlus(JOIS)

| C. DOCUMENTS CONSIDERED TO BE RELEVANT | | |
|---|---|---|
| Category* | Citation of document, with indication, where appropriate, of the relevant passages | Relevant to claim No. |
| A | JP 2002-162353 A  (Denso Corp.),<br>07 June, 2002 (07.06.02),<br>Par. Nos. [0032] to [0064]; Figs. 1 to 3 | 1-21 |
| A | US 5036861 A  (SEMBROWICH, W.L.),<br>06 August, 1991 (06.08.91),<br>Column 2, line 21 to column 4, line 57; Figs 1 to 5 | 1-21 |
| P,A | JP 2005-246054 A  (Sysmex Corp.),<br>15 September, 2005 (15.09.05),<br>Par. Nos. [0036] to [0080]; Figs. 1 to 10 | 1-21 |
| A | JP 63-135131 A  (Hitachi, Ltd.),<br>07 June, 1988 (07.06.88),<br>Page 2, lower left column, line 13 to page 4, lower left column, line 13; Fig. 2 | 1-21 |

| ☒ Further documents are listed in the continuation of Box C. | ☒ See patent family annex. |
|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>   25 January, 2006 (25.01.06) | Date of mailing of the international search report<br>   07 February, 2006 (07.02.06) |
|---|---|
| Name and mailing address of the ISA/<br>   Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/300121

C (Continuation). DOCUMENTS CONSIDERED TO BE RELEVANT

| Category* | Citation of document, with indication, where appropriate, of the relevant passages | Relevant to claim No. |
|---|---|---|
| A | JP 2003-514244 A (Spectrx, Inc.),<br>15 April, 2003 (15.04.03),<br>Par. Nos. [0023] to [0060]; Figs. 2 to 11 | 1-21 |

Form PCT/ISA/210 (continuation of second sheet) (April 2005)

<table>
<tr><td colspan="3" align="center"><b>INTERNATIONAL SEARCH REPORT</b><br>Information on patent family members</td><td>International application No.<br><br>PCT/JP2006/300121</td></tr>
</table>

```
JP  2002-162353 A      2002.06.07      (Family: none)

US  5036861 A          1991.08.06      (Family: none)

JP  2005-246054 A      2005.09.15      CN  1650795 A          2005.08.10
                                       EP  1561418 A1         2005.08.10
                                       US  2005/169799 A1     2005.08.04

JP  63-135131 A        1988.06.07      (Family: none)

JP  2003-514244 A      2003.04.15      AU  1778601 A          2001.05.30
                                       BR  15716 A            2003.05.06
                                       CA  2390893 A1         2001.05.25
                                       EP  1229825 A1         2002.08.14
                                       WO  2001/35820 A1      2001.05.25
```

Form PCT/ISA/210 (patent family annex) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9600110 A